# EUROPEAN PATENT APPLICATION

(11) **EP 4 679 232 A1**
(43) Date of publication of application: **14.01.2026**
(21) Application number: 25188782.4
(22) Date of filing: 10.07.2025
(51) Int. Cl.: G06F 3/01, A61B 5/00, A61B 5/11, A63B 24/00, H04R 1/10, H04M 1/72412, G06F 3/16

(54) **PROVIDING MOVEMENT DYNAMICS ESTIMATIONS**

(30) Priority: 11.07.2024 US 202463670092 P; 09.05.2025 US 202519204471
(71) Applicant: Apple Inc., Cupertino, CA 95014 (US)
(72) Inventor: MURGAI, Prateek, Cupertino, 95014 (US)
(74) Representative: COPA Copenhagen Patents

(57) **Abstract**

Aspects of the subject technology obtain sensor data from microphone and or movement sensors disposed in a device on each side of a user's body. The sensor data is used to provide running dynamics estimations including estimations involving differences between movement characteristics of the user's right side of the body versus the user's left side of the body. The estimations can further be used to provide feedback to the user regarding running style, running routes, and injury information.

## Description

### TECHNICAL FIELD

The present description relates generally to electronic devices, including, for example, using sensors in portable electronic devices to analyze motion characteristics.

### BACKGROUND

Headphones are an audio device that includes a pair of speakers, each of which is placed proximate to a user's ears. The pair of speakers of the headphones are normally connected (e.g., wired or wirelessly connected) to a separate playback device, such as a media playback device, which drives each of the speakers with an audio signal in order to produce sound (e.g., music). Some headphones can include audio input sensors (e.g., microphones), for example, so that a user's voice can be captured and relayed back to the playback device. Headphones can include other sensors.

### BRIEF DESCRIPTION OF THE DRAWINGS

Certain features of the subject technology are set forth in the appended claims. However, for purpose of explanation, several embodiments of the subject technology are set forth in the following figures.
FIG. 1 illustrates a diagram of an example electronic device and system that may implement aspects of the subject technology in accordance with one or more implementations.
FIG. 2 illustrates a block diagram of an audio signal processing architecture for an electronic device in accordance with one or more implementations.
FIG. 3 illustrates a data flow diagram for a block that performs movement dynamics estimation in accordance with one or more implementations.
FIG. 4 illustrates a data flow diagram for a block that performs movement dynamics estimation in accordance with one or more implementations.
FIG. 5 illustrates a data flow diagram for a block that performs sensor weighting and/or encoding based on sensed inputs.
FIG. 6 illustrates a flow diagram of an example process for performing movement dynamics estimation, in accordance with one or more implementations.
FIG. 7 illustrates an example user interface for providing information regarding various movement dynamics estimations over various time periods, in accordance with one or more embodiments.
FIG. 8 illustrates an example user interface for providing information regarding various movement dynamics estimations over a workout session, in accordance with one or more embodiments.
FIG. 9 illustrates an example electronic system with which aspects of the subject technology may be implemented in accordance with one or more implementations.

### DETAILED DESCRIPTION

The detailed description set forth below is intended as a description of various configurations of the subject technology and is not intended to represent the only configurations in which the subject technology can be practiced. The appended drawings are incorporated herein and constitute a part of the detailed description. The detailed description includes specific details for the purpose of providing a thorough understanding of the subject technology. However, the subject technology is not limited to the specific details set forth herein and can be practiced using one or more other implementations. In one or more implementations, structures and components are shown in block diagram form in order to avoid obscuring the concepts of the subject technology.

Some accessory devices, such as headphones include components designed to be disposed on either side of a user's body. Headphones may also include, in addition to speakers, microphones, inertial measurement units (IMUs), or other motion sensors, bone conduction devices, and so forth. Other accessory devices, such as glasses, head mounted displays, head bands, wrist bands, or purpose-built devices, may similarly have components disposed on either side of a user's body. The subject technology utilizes sensor input from sensors disposed on opposing sides of a user's body, analyzes the sensor input, and provides information related to a user's self-propelled movement, such as walking, jogging, or running. Because the information is provided from either side of the user's body, the result of such analysis can include information regarding stride length, gait, cadence, ground contact time, vertical oscillation, and so forth. Due to utilizing sensors on opposing sides of the user's body, these movement characteristics can be predicted and further analyzed for side-to-side imbalances. Such imbalances, for example, may be due to injury or bad running/walking form/habits. Whatever the cause for imbalance, because they can be measured, they can be addressed for correction.

For the sake of simplicity, the measurements gathered from the dual zoned sensors described herein are called, in general, running dynamics estimations. It should be appreciated that these measurements (or similar measurements from either side of the body for other activities) may be useful in contexts besides running, such as jogging, walking, cross-country skiing, sprinting, roller-skating, ice-skating, swimming, cycling, and so forth. Thus, a reference herein to running and/or running dynamics should be understood as not being limited to relating to the activity of running, but to any suitable movement characteristic. Therefore, the term running dynamics estimations as used herein may be understood to include any movement dynamics estimations, unless otherwise noted.

Running dynamics may be used to gauge performance. For competitors, running dynamics may be especially valuable to help track performance, improve performance, and prepare for specific events and/or competitions. These metrics are also useful to casual exercises who are trying to stay active as they can point towards issues with gait, issues with one foot versus the other foot, walking steadiness, and so forth. For an elderly person, for example, tracking such metrics can provide insight on measuring decline in steadiness or helping to pick proper footwear for better support. For seasoned performers, these metrics can inform areas of improvement while for casual performers, the metrics can inform areas to improve to prevent injuries. Those training for long distance running can use such metrics to their advantage because small imbalances in these metrics can accumulate over time to create bigger biomechanics issues if left unaddressed.

By way of explanation and without limitation, some of the running dynamics contemplated herein include cadence (total steps taken with both feet per minute), ground contact time (amount of time feet spend on the ground on each step), ground contact time balance (symmetry or asymmetry for ground contact time between the right and the left foot), stride length (the length of a half step), stride length imbalance (symmetry or asymmetry for stride length for the step of the right foot versus the left foot), vertical oscillation (the distance the foot is lifted for each step), and vertical oscillation imbalance (symmetry or asymmetry for vertical oscillation between the right foot and the left foot).

While some devices (e.g., a smart watch) may predict or estimate some of these metrics, using sensor input from both sides of the body can provide improved quality, alternate estimations which can be used when sensor data is noisy on the other devices, and additional metrics that a single-sided sensor is unable to provide. For example, the sensor signals used in the subject devices can be used to provide running dynamics estimations which can be combined with similar estimations from other devices, such as a smart watch, to offer a better estimation overall. Further, for example, the sensor signals on the subject devices may include microphone sensor inputs which are located within or adjacent to the ear canal and focused into the ear when worn by the user. These sensor signals may be much less susceptible to noise or drift issues than other signals, such as accelerometer signals used on a smart watch. Further still, for example, the sensor signals of the subject device are disposed on either side of the body, which provides data points distinct to each side, which may be used to predict running dynamics that a sensor device worn on one side of the body, such as a smart watch or phone, would be incapable of predicting because the missing signals, for example, cannot be fully or reliably approximated through a sensor device worn on one side of the body.

Aspects of the subject technology utilize headphone devices (e.g., earbuds) which include sensor devices disposed therein as a platform for obtaining signals for estimating running dynamics. In some implementations, the earbuds may include accelerometer sensors, for example, inertial measurement units (IMUs) in each bud, so that when worn by a user are disposed at each ear. In some implementations, the earbuds may include microphone sensors, such as in-ear microphone sensors disposed in each bud, so that when worn by a user are disposed at each ear, either in the ear canal or disposed adjacent to the ear canal, which in both cases may be within an air space or acoustical chamber with the ear canal. In some implementations, the earbuds may include both accelerometer sensors and microphone sensors.

Aspects of the subject technology calculate the running dynamics based on the sensor input and machine learning models trained to predict various running dynamics based on such sensor data. As a part of calculating the running dynamics, a footfall detection process can be used, and the footfalls can then be separated into the signals into distinct footfalls. These signals can be provided to a running dynamics estimation engine that subjects the signals to various processes, such as digital signal processing algorithms and/or machine learning models, such as classification and/or prediction models. These can provide as output, various estimates of running dynamics over time. In some aspects, the data received as sensor signals can be processed completely on the earbuds, in other aspects the data received as sensor signals can be partially processed on the earbuds and partially processed on one or more devices connected to the earbuds, in yet other aspects, the data received as sensor signals can be received on the earbuds and sent to one or more devices connected to the earbuds for processing.

Aspects of the subject technology can further utilize the determined running dynamics to provide feedback to the user. The feedback can include, for example, a user interface depicting graphs and values for the running dynamics over time. The feedback can indicate predictions for characteristics of the user's running style. For example, beyond the running dynamics noted above, the feedback can include providing to the user a prediction of whether the user is a heel striker, midfoot striker, or forefoot striker; a prediction of the users pronation (under pronation, over pronation, or normal pronation); or a prediction of an injury associated with the user (e.g., by comparing prior information with updated information). The feedback can also include feedback regarding the running surfaces and route taken by the user. For example, the feedback may include an estimate of the types of surfaces encountered on a run, such as an indication that a running route included 30% dirt or grass running and 70% pavement running. Depending on a selected training program, the user can be warned that they are running too much on pavement or too much on dirt or grass (or vice versa). In some aspects, a user can provide as inputs, a desired distribution of grass/dirt and pavement running, a desired length of route, and start and endpoints, and a route can be created and suggested by the device. In some aspects, a user can input running history events (such as their past running cycles) and compare current performance with past performance and receive feedback on the performance aspects. In some aspects, a user can select from provided historic running events (such as past Marathons) and the user can run those routes.

Aspects of the subject technology can provide real-time or near real-time feedback for some events, such as real-time warnings when a decrease in running form is detected. Other feedback can be determined and provided later.

The subject technology advantageously analyzes and provides information to users regarding running dynamics and other derived indicators from a running session. The subject technology can further provide running resources to users for enhancing running sessions such as mapping, running surface information, personal history comparisons, and historic running events. The subject technology can further provide both running style recommendations and product recommendations based on the running style, such as recommendations for footwear that supports the user's pronation and foot strike characteristics.

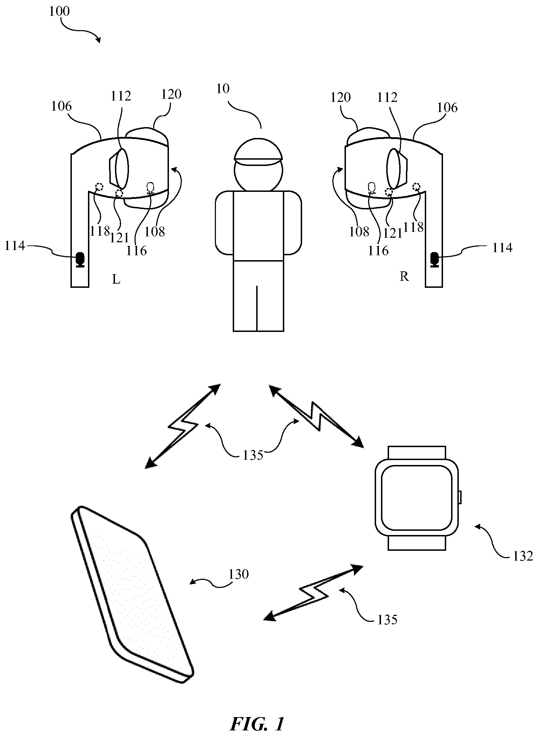

FIG. 1 illustrates an example electronic device and system in accordance with one or more implementations. Not all of the depicted components may be used in all implementations, however, and one or more implementations may include additional or different components than those shown in the figure. Variations in the arrangement and type of the components may be made without departing from the spirit or scope of the claims as set forth herein. Additional components, different components, or fewer components may be provided.

In the example of FIG. 1, electronic device 100 has been implemented using a housing 106 that is sufficiently small to be portable and carried or worn by a user. Specifically, FIG. 1 illustrates an example of the electronic device 100 implemented as an earbud or pair of earbuds. In this example, the housing 106 is shaped for seating in the user's concha and/or aperture of the ear canal and for interfacing with the user's ear canal. An optional sealing member 120 may interface with the housing 106 to provide a sealing effect between the electronic device 100 and the user's ear canal. The sealing member 120 may be made of any suitable material, such as silicone, plastic, rubber, foam, a polymer, a composite thereof, or combinations thereof.

In one or more other implementations, the earbud of FIG. 1 may be used in conjunction with another electronic device 130 or 132, such as a smartphone or tablet computer or smart watch to which microphone signals received by microphones 114 and/or 116) and/or sensors 118 and/or sensors 121 are transmitted to. The other electronic device 130 or 132 may also provide audio output signals for the speaker 112 to the electronic device 100. In one or more implementations, the earbud of FIG. 1 may include processing circuitry that performs one or more of the operations described herein.

In the example of FIG. 1, housing 106 includes openings 108. For example, openings 108 may form one or more respective ports for one or more respective audio components. In the example of FIG. 1, the electronic device 100 includes an opening 108 that forms a speaker port for a speaker 112 disposed within the housing 106. Other openings may include vents or port openings for the microphones 114 and/or 116. The openings for the microphones 114 may be disposed on an external surface of the housing 106 and the opening for the microphone 116 may be disposed on an internal surface of the housing 106 such that it is disposed within the opening 108 for the speaker 112. The microphone 116 may be positioned such that it is placed internal to or at the cusp of a user's ear canal or in an acoustic chamber or acoustically sealed chamber including the user's ear canal. Although two microphones are depicted, it should be understood that the electronic device 100 may include additional microphones. For example, in some implementations, the headphones may also provide active noise cancellation (ANC), which may also utilize a reference microphone disposed on an externally facing surface of the electronic device 100 when a user is wearing the headphones to measure the ambient noise to provide an anti-noise signal or a passthrough signal, depending on the user's settings.

In various implementations, the housing may also include fewer openings or additional openings. Additional openings may include openings for one or more additional audio input devices (e.g., microphones), one or more pressure sensors, one or more light sources, one or more light sensors, or other components that receive or provide signals from or to the environment external to the housing 106. Openings such as openings 108 may be open ports or may be completely or partially covered with a permeable membrane or a mesh structure that allows air and/or sound to pass through the openings.

The housing 106, which may sometimes be referred to as a case, may be formed of plastic, glass, ceramics, fiber composites, metal (e.g., stainless steel, aluminum, etc.), other suitable materials, or a combination of any two or more of these materials. The electronic device 100 also includes additional components such as processing circuitry (e.g., one or more processors), memory, a power source such as a battery, communications circuitry, and the like.

In one or more use cases, one or more of the speakers 112 may generate a speaker output based, for example, on a downlink communications signal or a device-generated or streaming audio signal. In one or more implementations, the speaker(s) 112 may be driven by an output downlink signal that includes far-end acoustic signal components from a remote device, such as the electronic device 130 or 132.

Aspects of the subject technology described herein may be performed by one or more processors of the electronic device 100 of FIG. 1 and/or may be performed by a processor inside the electronic device 130 and/or 132, upon receiving the microphone signals and/or other signals from a wired or wireless data communication link 135 with the electronic device 100 and/or with each other. The electronic device 100 in the example of FIG. 1 may include communications circuitry for communicating with one or more other electronic devices 130 and/or 132 via the wired or wireless data communication link 135.

In use, microphones 116, sensor 118, and/or sensor 121 may receive sensor inputs. The microphones 116 may receive inputs, for example, propagating from a user's foot, through the body, and to the ear. The sensor 118 may receive inputs related to the motion characteristics of the electronic device 100. The sensor 121 may receive inputs related to motion characteristics associated with the user speaking, such as vocal cord movement and/or jaw movement. The earbuds constituting the electronic device 100 of FIG. 1 may include a left earbud L and a right earbud R for use in a user's left and right ears, respectively.

In the example of FIG. 1, the electronic device 100 includes a speaker 112, a bottom microphone (e.g., microphone 114) that is located in or near an end portion of the housing 106 of the earbud where it is the closest microphone to the user's mouth, and an error microphone 116 that senses the sound at or near the user's eardrum (e.g., in the user's ear canal). In the example of FIG. 1, the microphone 116 may be in a position and orientation to receive an output from the speaker 112 and/or one or more ambient sounds (e.g., ambient noise, voices of people other than the user of the electronic device 100, a voice of the user of the electronic device 100, or the like) that leak past the housing 106 and/or sealing member 120 to reach the microphone 116. Although not pictured, a top microphone may be provided whose sound sensitive surface faces a direction that is opposite the eardrum of the user when the earbud is worn.

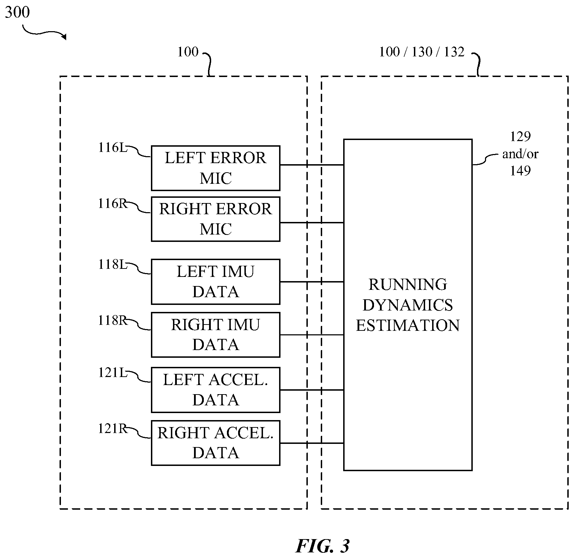

In one or more implementations, when the electronic device 100 is implemented as earbuds as in FIG. 1, the electronic device 100 may include a battery device, one or more processors, and/or a communication interface (see FIG. 3). The processors of the earbud may be a digital signal processing chip that processes an audio signal(s) (e.g., microphone signal(s)) from at least one of the microphones 114 and/or 116 and signals (e.g., IMU signals) from the sensors 118 and signals (e.g., voice accelerometer signals) from the sensors 121. The communication interface may include a Bluetooth^{™} receiver and transmitter to communicate the signals from the microphones 114 and 116 and/or sensors 118 and/or sensors 121 in both directions (uplink and downlink), with an external electronic device 130 and/or 132. Further, the earbuds may be wired earbuds or untethered wireless earbuds that communicate with each other and with the external electronic device 130 and/or 132 via Bluetooth^{™} signals.

In some aspects, the electronic device 100 may be communicatively coupled to the electronic device 130 and/or 132, such as demonstrated above via a wireless connection. In other aspects, the electronic device 100 may be communicatively coupled with the electronic device 130 and/or 132 via other methods. For example, both devices may couple via a wired connection. In this case, one end of the wired connection may be (e.g., fixedly) connected to the electronic device 100, while another end may have a connector, such as a media jack or a universal serial bus (USB) connector, which plugs into a socket of the electronic device 130 and/or 132. Once connected, the electronic device 130 and/or 132 may be configured to drive one or more speakers of the electronic device 100 with one or more audio signals. For instance, the electronic device 130 and/or 132 may transmit the audio signals as digital audio (e.g., PCM digital audio). In another aspect, the audio may be transmitted in analog format.

The electronic device 130 and/or 132 may be any electronic device (e.g., with electronic components, such as one or more processors, memory, etc.) that is capable of streaming audio content, in any format, such as stereo audio signals, for playback (e.g., via one or more speakers integrated within the source device and/or via one or more output devices, as described herein). For example, the source device may be a desktop computer, a laptop computer, a digital media player, etc. In one aspect, the device may be a portable electronic device (e.g., being handheld operable), such as a tablet computer, a smart phone, etc. In another aspect, the source device may be a wearable device (e.g., a device that is designed to be worn on (e.g., attached to clothing and/or a body of) a user, such as a smart watch. In some aspects, the electronic device 130 may include an interface and programming contained in memory which when executed by the one or more processors performs a hearing test utilizing the electronic device 100.

The discussion herein describes the electronic device 100 in a use case as being a pair of earbuds, for the sake of simplicity. It should be understood, however, that the electronic device may be any suitable device with sensors disposed on either side of the body, such as any suitable wearable devices which may be worn or attached on opposing sides of an ambulatory body. This may include two separate devices having sensors which are worn on each side, which can communicate with each other and/or with a third device, such as a smartphone or smartwatch for storing and/or processing data using the processes and techniques discussed herein, or a single device having sensors that are disposed on each side of the body when worn, which can communicate with another device, such as a smartphone or smartwatch for storing and/or processing data. For example, in some embodiments, the electronic device 100 may be implemented as over the ear or on the ear headphones, glasses, head mounted displays, head bands, wrist bands, or purpose-built devices, each with sensor components disposed on either side of a user's body. Moreover, other sensors may be suitable to detect one or more similar signals for the detection and processing of running dynamics.

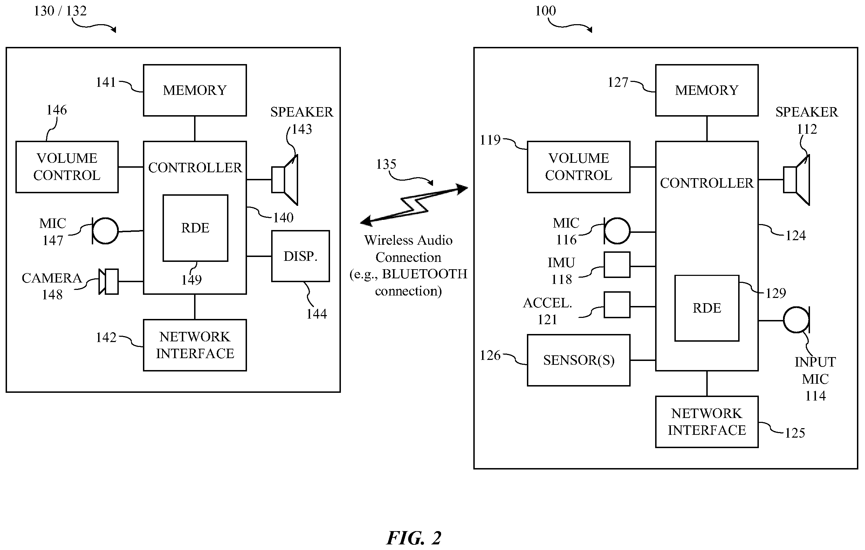

FIG. 2 shows a block diagram of the electronic device 130 and/or 132 and of the electronic device 100 that are wirelessly coupled together according to one aspect. The electronic device 130 and/or 132 includes a controller 140, memory 141, a network interface 142, a speaker 143, a display 144, a microphone 147, a camera 148, and a volume control 146. In one aspect, the source device may include more or less elements as described herein. For instance, the device may include several speakers and/or microphones or may not include the camera or the display.

According to one aspect, the electronic device 100 includes a controller 124, a network interface 125, an audio output device, such as speaker 112, microphones 114 and 116, sensor 118 (e.g., an IMU configured to detect movement of the electronic device 100), sensor 121 (e.g., an accelerometer configured to detect voice input from a user of the electronic device 100), one or more (other) sensors 126, a volume control 119, and memory 127. In one aspect, the device may include more or less elements. For example, the electronic device 100 may include one or more additional speakers, and/or may include one or more additional microphones.

The microphone 114 may be an input microphone, such as a vocal microphone and microphone 116 may be an error microphone whose primary function is for ANC, but which can also be used for processes described herein. A reference microphone (not shown) may also be included in the electronic device 100 for ANC. In the case of an earbuds or headphones device, the microphone 116 may sense sound inside the user's ear when the electronic device 100 is positioned on (or in) the user's ear.

The sensor 118 (e.g., IMU) may be designed to produce motion data that indicates (changes in) the position and/or orientation of the output device. In another example, the sensors may include an accelerometer that may be arranged and configured to receive (detect or sense) vibrations that are produced while a user (e.g., who may be wearing the output device) is in motion and produce an accelerometer signal that represents (or contains) motion vibrations. Specifically, the accelerometer is configured to sense bone conduction vibrations that are transmitted through the body when running or walking. For example, when the electronic device is a wireless headset, the accelerometer may be positioned anywhere on or within the headphone, which may touch a portion of the user's body in order to sense vibrations. The sensor 121 (e.g., an accelerometer) may be designed to produce motion data that indicates that a user of the electronic device 100 is speaking. Specifically, it may be tuned to detect motion of the user's jaw and/or vibrations of the user's vocal cords. The sensors 126 may include one or more other sensors that are designed to produce sensor data. For instance, the sensors may include a proximity sensor or light sensor, for determining whether the user is wearing the electronic device 100.

The controller 124 may be a special-purpose processor such as an application-specific integrated circuit (ASIC), a general-purpose microprocessor, a field-programmable gate array (FPGA), a digital signal controller, or a set of hardware logic structures (e.g., filters, arithmetic logic units, and dedicated state machines). In one aspect, controller 124 may be configured to perform digital signal processing in association with the running dynamics estimation (RDE). Microphone signals and accelerometers output signals and the controller 124 can obtain those signals and perform one or more steps leading to the performing the RDE. For example, the signals can be pre-processed using digital signal processing to remove noise and isolate the footfalls of the user which are picked up by each sensor. The sensors, such as the microphone 116, the sensor 118, and the sensor 121, may be subject to various aggressors which can cause noise or error in the signals. Aggressors may include, for example, breathing (respiration sounds), playback sounds (e.g.., from speaker 112), fit changes while running or moving, handling noise, sensor drift issues, wind noise, heartbeat noise, external or ambient sounds, and so forth. The footfall information can be extracted from the sensor input and isolated for analysis. Audio processing filters (e.g., high pass, low pass, band pass, etc.) can be applied and/or other digital signal processing used to clean the signals before and/or after performing the isolation of the footfall data. Further, weighting schemes can be used as discussed in greater detail below to place more emphasis on the input from one sensor versus another when certain aggressors are more prevalent than other aggressors.

Footfall data, for example, can include sensor input information for a user's left foot contact with the ground sensed by the user's left-side sensors and the user's right-side sensors at a lesser intensity than the left-side sensors, and vice versa. The isolated footfall data can further be processed to separate the footfalls into right foot and left foot footfalls. The isolated footfall data can be provided to a local RDE processing block 129 for performing some or all of the RDE processing. In some instances, some or all of the processing of the sensor signals may be sent to another device, such as the electronic device 130 and/or 132, to process at an RDE processing block on one or more of such devices. This may be done, at least in part, for example, to save processing and power (battery) resources on the electronic device 100.

The controller 124 may also be configured to perform other functions, such as (other) audio signal processing operations and/or networking operations. For instance, the controller may be configured to obtain (or receive) audio data (as an analog or digital audio signal) that includes audio content, such as music for playback through the speaker 112. In some aspects, the controller may obtain audio data from memory 127, or the controller may obtain audio data from another device, such as the source device via the network interface 125. For instance, the output device may stream an audio signal from the source device (e.g., via the Bluetooth^{™} connection) for playback through the speaker 112. The audio signal may be a signal input audio channel (e.g., mono). In another aspect, the controller may obtain two or more input audio channels (e.g., stereo) for output through two or more speakers.

The volume control 119 may be configured to adjust a volume level of sound output of the electronic device 100 in response to receiving a user-adjustment (e.g., user input) at the control. For instance, upon receiving user input, the control 119 may adjust the (e.g., overall) volume of sound output by the (e.g., speaker 112 of the) electronic device 100. In some aspects, the volume control 119 may be used to adjust the volume setting at the electronic device 130. In one aspect, the volume control may be a "physical" volume control that may be a dedicated volume input control, such as one or more buttons, a rotatable knob, or a physical slider. For instance, the volume control 146 may include at least two buttons, a "volume up" button, which may be configured to perform a stepwise increase to the volume each time it is pressed by the user, and a "volume down" button that may be configured to perform a stepwise decrease to the volume each time it is pressed by the user. In some aspects, the volume control may be any type of physical input device that can adjust the volume level.

In the electronic device 130 and/or 132, the controller 140 may be a special-purpose processor such as an application-specific integrated circuit (ASIC), a general purpose microprocessor, a field-programmable gate array (FPGA), a digital signal controller, or a set of hardware logic structures (e.g., filters, arithmetic logic units, and dedicated state machines). The controller 140 is configured to perform operations for performing running dynamics estimation (RDE) and processes associated with an RDE estimate, such as described in further detail below. The controller 140 may also be configured to perform other processing, such as audio signal processing operations, and/or networking operations. The controller 140 may have disposed therein hardware, logic blocks, instructions, or the like which provide aspects of the running dynamics estimation (RDE), which together are referred to as RDE block 149.

The memory 141 may be any type of non-transitory machine-readable storage medium. Examples may include read-only memory, random-access memory, CD-ROMS, DVDs, magnetic tape, optical data storage devices, flash memory devices, and phase change memory.

The camera 148 may be a complementary metal-oxide-semiconductor (CMOS) image sensor that is capable of capturing digital images including image data that represent a field of view of the camera, where the field of view includes a scene of an environment in which the electronic device 130 is located. In some aspects, the camera may be a charged-coupled device (CCD) camera type. The camera is configured to capture still digital images and/or video that is represented by a series of digital images. In one aspect, the camera may be positioned anywhere about/on the electronic device 130. In some aspects, the electronic device 130 may include multiple cameras (e.g., where each camera may have a different field of view).

The microphone 147 may be any type of microphone (e.g., a differential pressure gradient micro-electro-mechanical system (MEMS) microphone) that is configured to convert acoustical energy caused by sound waves propagating in an acoustic environment into a microphone signal. In some aspects, the microphone may be an "external" (or reference) microphone that is arranged to capture sound from the acoustic environment.

The speaker 143 may be an electrodynamic driver that may be specifically designed for sound output at certain frequency bands, such as a woofer, tweeter, or midrange driver, for example. In one aspect, the speaker 143 may be a "full-range" (or "full-band") electrodynamic driver that reproduces as much of an audible frequency range as possible.

The display 144 is designed to present (or display) digital images or videos of video (or image) data. In one aspect, the display may use liquid crystal display (LCD) technology, light emitting polymer display (LPD) technology, or light emitting diode (LED) technology, although other display technologies may be used in other aspects. In some aspects, the display may be a touch-sensitive display screen that is configured to sense user input as touches or taps on the screen, and in response produce one or more control signals. In some aspects, the display may use any touch sensing technologies, including but not limited to capacitive, resistive, infrared, and surface acoustic wave technologies.

The volume control 146 is configured to adjust a volume level of sound output of the electronic device 130 and/or 132 in response to receiving a user-adjustment (e.g., user input) at the control. In one aspect, the volume control may be a "master" volume control that is configured to control the overall volume level (e.g., sound output level of the speaker 143) of the electronic device 130 and/or 132. In another aspect, when the electronic device 130 and/or 132 is communicatively coupled with the electronic device 100 in order to stream audio content to the electronic device 100, for playback, the volume control may control the overall volume level of the electronic device 100 (as well). In one aspect, the volume level output on the electronic device 100 for a hearing test process or application may override the setting provided by way of the volume control 146, for example, by a user. In one aspect, the volume control may be a "physical" volume control that may be a dedicated volume input control, such as one or more buttons, a rotatable knob, or a physical slider. For instance, the volume control 146 may include at least two buttons, a "volume up" button, which may be configured to perform a stepwise increase to the volume each time it is pressed by the user, and a "volume down" button that may be configured to perform a stepwise decrease to the volume each time it is pressed by the user. In some aspects, the volume control may be any type of physical input device that can adjust the volume level.

FIG. 3 illustrates a data flow diagram 300 between the sensors of the electronic device 100 and a running dynamics estimation (RDE) block 129 and/or 149. Although the discussion of the data flow diagram 300 proceeds below describing the data flow from block to block, it should be understood that flow can be performed in parallel as appropriate or may flow out of the order described where the associated processes allow for the data flow to differ.

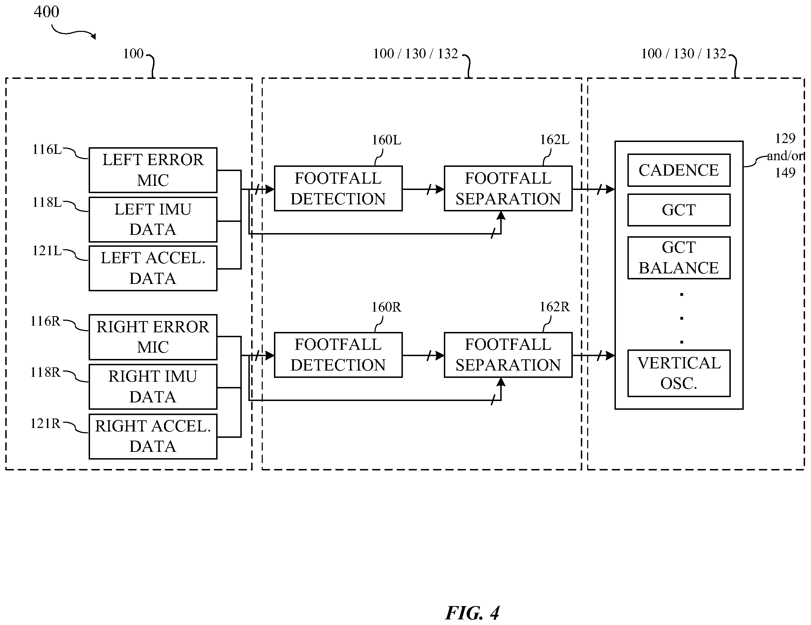

As described below, the RDE block 129 and/or 149 may include running dynamics estimation functionality utilizing digital signal processing techniques, such as filtering, Fourier transforms, and wave analysis to analyse sensor data inputs for estimating running metrics associated with a user being sensed by the sensors. The RDE block 129 and/or 149 may provide, for example, outputs including characterizations of a user's running activity based on the sensor inputs utilizing the digital signal processing techniques. These outputs are described in further detail with respect to FIG. 4. These techniques can be understood as corresponding to non-AI based processes to obtain digital and/or analog sensor data from the microphones, IMU, and accelerometers of each bud to indicate usage and environmental conditions.

Sensor inputs, including a left error mic 116L, a right error mic 116R, a left IMU 118L, a right IMU 118R, a left voice accelerometer 121L, and a right voice accelerometer 121R, from sensors can be fed to the RDE block 129 and/or 149 for processing and providing running dynamics estimations. The RDE block 129 and/or 149 can include processes implementing one or more learning-based and/or non-learning-based models for perceiving, synthesizing, and inferring information. Persons skilled in the art will appreciate that the RDE block 129 and/or 149 can include any suitable number of RDE processes to generate output characterizations (e.g., cadence, ground contact time, ground contact time balance, vertical oscillation, gait, and so forth) based on the sensor inputs. In some embodiments, the RDE block 129 and/or 149 may optionally elect to use a lightweight engine instead of a multi-faceted process at the RDE block 129 and/or 149 to process data in situations where only one modality of data is being processed. The lightweight engine 108 may, for example, be a non-learning network.

Persons of ordinary skill in the art will appreciate that processes implemented in the RDE block 129 and/or 149 may include any suitable machine learning models that are well-known or widely available such as regression techniques, classification techniques, neural networks, and deep learning networks. For instance, the RDE block 129 and/or 149 can include neural networks such as Artificial Neural Network (ANN), Convolutional Neural Network (CNN), Recurrent Neural Network (RNN), Adversarial Network (GAN), Reinforcement Learning Model (RLM), Encoder/Decoder Networks, and/or Transformer-Based Models (e.g., Bidirectional Encoder Representations from Transformers (BERT), Generative Pre-trained Transformer (GPT), and/or a multi-modal large language model (LLM)). Additionally or alternatively, persons of ordinary skill in the art will appreciate that the RDE block 129 and/or 149 can include any suitable non-learning processes such as rule-based systems, heuristics, decision trees, knowledge-based systems, statistical or stochastic systems, and expert systems.

In instances where the RDE block 129 and/or 149 implements a machine-learning based model, the processes of the RDE block 129 and/or 149 can be trained to disambiguate between footfalls and to characterize running dynamics using one or more well-known or widely available training techniques such as supervised learning, semi-supervised learning, unsupervised learning, and/or reinforcement learning techniques. The training data can include known route and/or surface information, known physical characteristics of the training user data, and location information correlated to footfall information. Additional training techniques and considerations data are further described below.

In instances where the RDE block 129 and/or 149 is a non-learning-based system, the RDE block 129 and/or 149 can use a predefined set of rules or a predefined structure to make decisions based on the inputs that the block sees. For example, the RDE block 129 and/or 149 can process each signal in parallel as the data is streamed via the signal source and compare interaction points between signals to determine a pattern, for example, indicating cadence or footfall, a pattern, for example, indicating whether a runner is running on a hard surface or a natural or springy surface, or a pattern, for example, that estimates a vertical oscillation metric associated with running. It can then prepare characterizations using the various signals and their interactions

The sensor inputs from the electronic device 100 can include a left error mic 116L, a right error mic 116R, a left IMU 118L, a right IMU 118R, a left voice accelerometer 121L, and a right voice accelerometer 121R. In some implementations, one or more of the IMUs may be omitted (or may include just an accelerometer sensor type), one or more of the error mics may be omitted, and/or one or more of the vocal accelerometers may be omitted. The sensor inputs may each be provided to a running dynamics estimation (RDE) block 129 and/or 149. For example, the sensor inputs may be provided to the RDE block 129 of the electronic device 100 and/or the RDE block 149 of the electronic device 130 and/or 132. In some embodiments, some of the sensor inputs can be provided to the RDE block 129 and others can be provided to the RDE block 149. For example, the left error mic 116L signal and right error mic 116R signal can be provided to the RDE block 129 of the electronic device 100 and the left IMU 118L signal and right IMU signal 118R can be provided to the RDE block 149 of the electronic device 130 and/or 132, or vice versa. In some embodiments, the sensor inputs can be provided to both the RDE block 129 and the RDE block 149. The sensor inputs can be sent at different times. For example, the sensor inputs can initially be sent to the electronic device 100 in real time and sensor inputs can be sent to the electronic device 130 and/or 132 periodically, such as every 0.5 to 15 minutes or every 15 minutes to 60 minutes, or sent at a prescribed time or condition, such as at the end of a running session, time of day, status of device (e.g., in case and charging), or when a battery level of the electronic device 100 meets a threshold.

As noted in FIG. 3, the sensor inputs are provided by the electronic device 100. The RDE 129 and/or 149 can be provided by the electronic device 100, the electronic device 130, or the electronic device 132 (or a combination thereof).

The RDE block 129 and/or 149 can receive the signals and perform the RDE on the signals. The RDE can, for example, supply the signals to a machine learning model or perform digital signal processing (or a combination of both) to determine measurements indicating various characteristics of the user's gait.

In one aspect, user running attributes may be determined such as cadence, ground contact time imbalance, ground contact time, and vertical oscillation. No other input may be needed from a user to obtain the data, however, if the user desires, setting target ranges may be performed manually or automatically based on historical data, and if the user goes outside of these ranges, then the user may be notified, for example, by an audio output of the electronic device 100 and/or by an audio, vibratory, or visual output on the electronic device 130 and/or 132.

In another aspect, user gait attributes may be determined such as over striding, heel striking, forefoot striking, or midfoot striking. For example, the RDE block 129 and/or 149 may use the user running attributes along with the signals (which may be de-noised or weighted as discussed below) to predict whether user is over striding or not, to predict whether user is a heel striker versus a forefoot or midfoot striker. In this aspect, if the user provides input that they are a heel striker and would like to transition to forefoot/midfoot striking, the RDE block 129 and/or 149 can be trained first using the natural gait of the user (for example heel striker) and then as the user tries to transition to a different kind of striking the system can provide feedback to the user based on current gait attribute predictions. For example, the system can provide feedback via an audio output to the electronic device 100 and/or by an audio, vibratory, or visual output on the electronic device 130 and/or 132, the feedback indicating the progress and/or status of the user's transition.

In another aspect, surface detection can be performed by the RDE block 129 and/or 149. This aspect may inform a user if the user is running too much on one detected surface versus another which could cause any further injury or issues. A user may provide input regarding past injury or current gait type, and/or goals for which surfaces the user would want to run. In such aspects, a training period may be used to train a model based on the user and the user's equipment such as shoe type. For example, a user may begin a session and provide input to the system that indicates whether the run is an asphalt run or dirt trail run. Over time, the system can then derive latent space information/embeddings about the surface using the left/right sensors. Once trained over one to two running sessions, the model can be used to predict how much time and/or distance of a running session is spent on asphalt or dirt. The model may also be used to predict transitions to different surfaces within a given run and correlate that information to GPS data to determine map information for the user or for the system in general to be used by the system for the purpose of suggesting running routes for other users with specific surface types.

In this aspect, the system can also notify the user that given the user's current gait analysis and past injuries for a given detected surface can be harmful to the recovery or may cause further injuries. For example, the system can provide a notification via an audio output to the electronic device 100 and/or by an audio, vibratory, or visual output on the electronic device 130 and/or 132, the notification indicating a warning with regard to the current gait and/or surface detection.

In a use case, the electronic device 100 can receive one or more signal pairs and provide the signal inputs to the RDE block 129. The RDE block 129 can perform a first analysis on the signals to determine one or more characteristics of the user's gate and trigger a response when one or more of the characteristics are out of a bound of acceptable values (e.g., beyond an acceptable threshold) for that respective characteristic. For example, if the stride length of the user is too short or too long (as compared to previous sessions), then the user could be notified during their running session for the purposes of suggesting a corrective action in real time or in near real time (e.g., during the session). The notification can come by the electronic device 100 or the electronic device 130 and/or 132. The one or more characteristics by the first analysis can be a subset of the available characteristics. Then, in a second analysis of the signals occurring at a later time (after being provided to the RDE block 149), the signals can be analyzed again at the RDE block 149 and the one or more characteristics determined again with more detail and/or by using different (e.g., more complex) machine learning models. In addition, the second analysis of the signals can include applying additional machine learning models and/or digital signal processing to estimate additional characteristics of the running session.

In another use case, a similar process can be undertaken as that described in the preceding paragraph, except the signals can be provided to the RDE block 149 of the electronic device 130 and/or 132 and a first analysis can be performed in near real time (e.g., depending on how often the signal data is provided from the electronic device 100). Then, the processing can be performed on the electronic device 130 and/or 132.

FIG. 4 illustrates a data flow diagram 400 between the sensors of the electronic device 100 and a running dynamics estimation (RDE) block 129 and/or 149. Although the discussion of the data flow diagram 400 proceeds below describing the data flow from block to block, it should be understood that flow can be performed in parallel as appropriate or may flow out of the order described where the associated processes allow for the data flow to differ.

The sensor data from the electronic device 100 may be the same as that discussed above with respect to FIG. 3. The RDE block 129 and/or 149 may likewise be the same as that discussed above with respect to FIG. 3. A signal processing data flow between the signals and the RDE block 129 and/or 149 may be used to reduce the data of the input signals and pre-process the signals to provide an abstraction of the signal data to the RDE block 129 and/or 149.

For the left side, the signals may be provided to a footfall detection block 160L which can reduce noise on the left error mic 116L signal, the left IMU 118L signal, and/or the left voice accelerometer 121L. For example, IMU drift can cause two identical footfalls at two different points in the running session to have different measurements. Reducing noise can help reduce the impact of IMU drift. Further, because the error mic may also be in use by ANC, then reducing noise in the error mic signal can help remove some of the noise associated with the ANC process. The footfall detection block 160L can extract the footfall information for the user, for example, by applying filtering to target certain frequency ranges, for example, where the footfall information would be detected. These frequency ranges can vary depending on which surface is being run on. Thus, the footfall detection may be a dynamic process that can analyze the signals for pattern matching footfall characteristics across the available frequency range and then apply a filter based on the frequency where the pattern occurred. Footfall detection block 160L may also be used to detect that the user has begun running to initiate the digital signal processing associated with performing the running dynamics estimations described herein.

Footfall detection block 160L may also take as inputs various sensor information from the opposite side device, for example, in this case, the electronic device 100 disposed on the right side of the user. The sensor information may be encoded sensor information, that is, the sensor information may be processed to simplify the representation of the sensor information using a suitable encoding and/or sampling technique. Likewise, the footfall detection block 160L may provide similar sensor information to the opposite electronic device 100, in this case, the electronic device 100 disposed on the right side of the user. The footfall detection block 160L can use the sensor information from the opposite side device to help correlate footfall information from the opposite side with the footfall information from the current side to provide a more accurate extrapolation of footfall detection.

Following the footfall detection block 160L, the resulting footfall data can be provided to the footfall separation block 162L, in addition to the original (and optionally noise reduced) signal. The footfall separation block 162L can analyze the data to separate footfalls into right footfalls and left footfalls. For example, for the footfall separation block 162L, because it is for processing the left side data, footfalls detected for the left foot may be more prominent in the sensor data than footfalls of the right foot.

At the same time, footfall detection block 160R and footfall separation block 162R may be executed in like manner as the footfall detection block 160L and footfall separation block 162L to provide footfall separation information to the RDE block 129 and/or 149.

The signal processing flow including the footfall detection block 160L and 160R and footfall separation block 162L and 162R may be executed by the electronic device 100 and/or 130 and/or 132. For example, this signal processing flow can also reduce a data size of the signal data so that transfer to the electronic device 130 and/or 132 may be more efficient and use less power than the preprocessed signals from the sensors.

The RDE block 129 and/or 149 illustrated in FIG. 4 also illustrates some of the running dynamics characteristics that can be estimated. As noted above, these can include, for example, one or more of cadence, ground contact time, ground contact time balance, stride length, stride length imbalance, vertical oscillation, or vertical oscillation imbalance, and so forth.

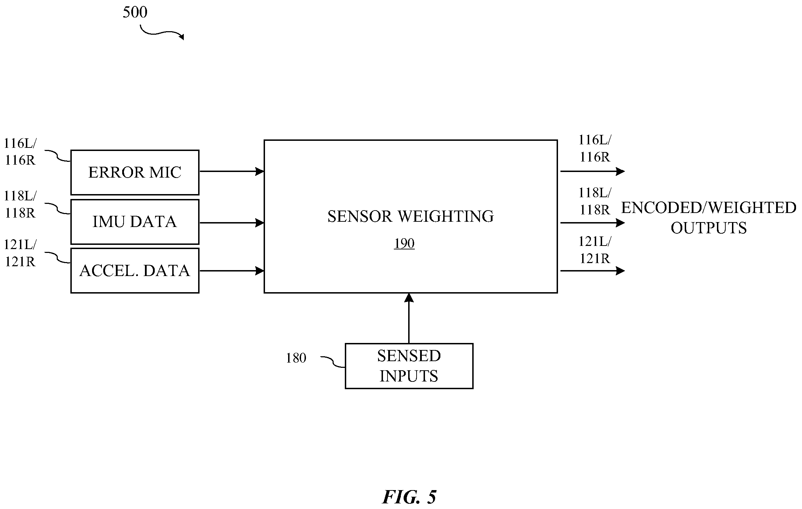

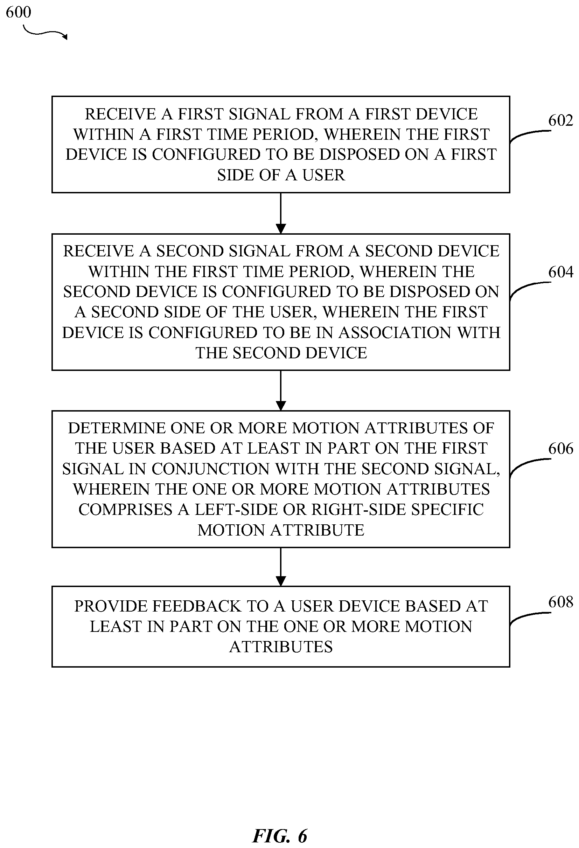

FIG. 5 illustrates a data flow diagram 500 between the sensors of the electronic device 100 and a sensor weighting block 190. Although the discussion of the data flow diagram 500 proceeds below describing the data flow from block to block, it should be understood that flow can be performed in parallel as appropriate or may flow out of the order described where the associated processes allow for the data flow to differ. The flow of the data flow diagram 500 may be applied to each of the sensor data discussed with respect to FIGS. 3 and 4 and also to the sensor data discussed below with respect to FIG. 6.

The sensor data from the electronic device 100 may be the same as that discussed above with respect to FIG. 3. The sensor weighting block 190 may take as inputs, the sensor data and sensed inputs 180 and provide weighted and/or encoded outputs corresponding to each of the same sensor data signals. Each side (left/right) may have its own sensor weighting block 190 or may use a common sensor weighting block 190. For example, the left error mic signal 116L, the left IMU data signal 118L, and the left voice accelerometer signal 121L may each be provided into the sensor weighting block 190 which may be provided by the controller 124 of the electronic device 100 and/or provided by the controller 140 of the electronic device 130/132. The sensor weighting block 190 may also take as input, sensed inputs 180 regarding the usage mode or environmental factors which may affect the weighting of the signals. The sensor weighting block 190 may use the input signals and the sensed inputs 180 and provide encoded and/or weighted outputs, each corresponding to the respective input signals.

The sensed inputs 180 may include inputs indicating a sensed physical environment for the device. For example, the sensed inputs 180 may indicate the mode of operation, the playback volume, the ambient sound pressure level, and/or the activity level. For example, a mode of operation of the electronic device 100 may correspond to ANC mode for noise cancellation, transparency mode for audio passthrough, or auto ANC for a dynamic blend of the two. If the mode of operation includes ANC mode, then the error microphone 116 may be weighted greater. However, when the mode of operation includes transparency mode, then the error microphone 116 may be weighted less. Similarly, if the playback volume of the electronic device 100 (for example, as controlled by the electronic device 130 and or 132) is high, making it more difficult to use the voice accelerometer and error microphones due to volume and residual echo, then the motion sensors and external microphones (e.g., microphone 114 and or an ambient microphone, which may be used instead or in addition to the error microphone 116) might be weighted higher. In contrast, as the ambient sound pressure level increases, any external microphones may be weighted less in proportion to the ambient sound pressure level. In the presence of a high degree of user activity, active motion sensors, the motion sensors might be very sensitive to activity of a user and make the signals noisy during an activity. Thus, when a high degree of user activity is occurring (e.g., intense running or walking), then lowering the weighting on the motion sensors may be performed, thereby lowering the weights on these signals during intense activity.

Adjusting the weightings can be performed by a rule based operation based on thresholds of the sensed inputs 180 which can turn on or turn off certain signals or provide linear or non-linear amplification or attenuation based on mapping of the sensed inputs 180 to various input ranges. For example, ambient sound pressure level value can drive the weights on some of the sensors like the error microphone and external microphones.

In some implementations, weighting can be also be determined using a trained machine learning model encoder which utilizes the signals and a vector of the sensed inputs 180 as inputs to the machine learning model and produces an encoded representation of the input signals as output signals from the sensor weighting block 190.

FIG. 6 illustrates a flow diagram of an example process 600 for utilizing running dynamics estimation (RDE) in accordance with some embodiments. One or more blocks (or operations) of the process flow diagram of FIG. 6 may be performed by one or more other components and other suitable devices. Further for explanatory purposes, the blocks are described herein as occurring in serial, or linearly. However, multiple blocks of the process 600 may occur in parallel. In addition, the blocks of the process need not be performed in the order shown and/or one or more blocks of the process 600 need not be performed and/or can be replaced by other operations.

At block 602, process 600 may include receiving a first signal from a first device, where the first device is configured to be disposed on a first side of a user. In some implementations, the first signal may include a signal from a first accelerometer or a first audio input. The audio inputs, for example, may correspond to error microphones or external microphones, as described above. The accelerometers may also include other motion data, such as provided by an inertial measurement unit (IMU). Additional signals may be used as the first signal, including voice accelerometer signals and other microphone signals. Further, the first signal may be weighted signals such as discussed above with respect to FIG. 5.

At block 604, process 600 may include receiving a second signal from a second device, where the second device is configured to be disposed on a second side of the user, where the first device is configured to be in association with the second device. In some implementations, the first signal may be from a first in-ear microphone and the second signal may be from a second in-ear microphone. Additional signals may be used as the second signal, including voice accelerometer signals and other microphone signals. Further, the second signal may be weighted signals such as discussed above with respect to FIG. 5.

The second signal can be received in the same time window as the first signal such that distinct consecutive footfalls within the time window may be obtained from the signals for each foot individually and for each foot in sequence with the other foot. Although, the first signal may be used primarily in conjunction with the foot that is on the side of the user as the location of the first signal and the second signal may be used primarily in conjunction with the foot that is on the side of the user as the location of the second signal, it should be appreciated that each signal may include footfall information for both feet. The first and second signal can be used in combination to determine movement/running characteristics regarding each foot and both feet together.

Some aspects may also include receiving a third signal from the first device, the third signal having the other of the signal from the first accelerometer or the first audio input, and receiving a fourth signal from the second device, the fourth signal having the other of the signal from the second accelerometer or the second audio input. In such aspects, the determining the one or more motion attributes of the user discussed at block 606 may be based further at least in part on the third signal and the fourth signal. Additional signals may be used as the third signal and/or fourth signal, including voice accelerometer signals and other microphone signals. Further, the third signal and fourth signal may be weighted signals such as discussed above with respect to FIG. 5.

Similar to that noted above with respect to the first and second signals, the third and fourth signals can be used in conjunction with the first and second signals and with each other within a same time window to determine footfall information from which to derive characteristics for each foot individually and for both feet together, for example, using the techniques described herein.

At block 606, process 600 may include determining one or more motion attributes of the user based at least in part on the first signal and the second signal (and optionally the third and fourth signals), where the one or more motion attributes comprises a left-side or right-side specific motion attribute. In some implementations, the process 600 may include estimating one or more of cadence, ground contact time, ground contact time balance, stride length, or vertical oscillation, based at least in part on the one or more motion attributes. In some aspects, estimation of ground contact time balance can be used to determine heel strike information corresponding to a left foot of the user and heel strike information corresponding to a right foot of the user. The left-side specific motion attribute can be used in association with the right-side specific motion attribute to determine the one or more motion attributes. For example, the one or more motion attributes can include a determination of left-side or right-side compensation.

Determining the one or more motion attributes can include analyzing each of the input signals within a period of time in conjunction with each other to derive information from the signals which are time aligned, and which can demonstrate within the respective input signal, for example, a first footfall followed by a second footfall from the opposite foot followed by a another footfall from the first foot, and so on, over the period of time. In addition to the footfalls themselves, the first signal, second signal, and optionally the third signal and/or fourth signal can further include sensed inputs that indicate a surface-type and other environmental factors, as well as running-style characteristics (e.g., foot strike information, cadence, ground contact time, ground contact time balance, stride length, and vertical oscillation).

The estimating may include calculating embeddings based at least in part on the one or more motion attributes and providing the embeddings to a classification model to determine the estimations. The classification model, for example, may correspond to one or more machine learning models trained on different motion types.

The estimating or determining may include providing the first signal and the second signal (and optionally the third and fourth signals) to a machine learning model trained to predict motion attributes based on input signals. Further, the estimating or determining may include receiving the one or more motion attributes from the machine learning model. For example, a machine learning model can be used to predict the motion attributes. In some implementations, each attribute may have its own machine learning model, while in other implementations, at least some of the attributes can be predicted by a combined machine learning model. In particular, it may not be feasible or possible to reliably determine such attributes based on signal analysis alone, but by utilizing a machine learning model, the attributes can be determined (e.g., estimated or predicted to a threshold degree of reliability) based on the training data to the model.

At block 608, process 600 may include providing feedback to a user device based at least in part on the one or more motion attributes (block 608). In some implementations, for example, the feedback includes an indication of detected right side or left side compensation. For example, the ground contact time balance can indicate that a user spends more time on the right foot versus the left foot, indicating that the right foot is being favored on account of some compensation to relieve the left foot. While such side-to-side compensation may relieve injury or pain temporarily, prolonged compensation may lead to adverse consequences, such as compensation injury to the other foot, for example.

In some implementations, for example, the feedback includes an estimation of a percentage of time spent running on hard surfaces versus soft surfaces. The type, frequencies, and amplitude of the signals can indicate whether the running is on a hard surface versus a soft surface. This information can be provided to the user to determine if the user is meeting their training goals. For example, running on softer surfaces may provide less wear on the joints of the user and so an encouragement may be made for the user to mix up the surface types being run on to reduce the risk of injury and/or wear. Whether the user wants to run on soft or hard surfaces, for example, can be used to correlate a shoe type with the type of running. For example, if the user indicates that they are trail runners, then a shoe with a stiffer sole may be more desirable, but if the user actually is running on harder surfaces, then the user can be recommended a softer soled shoe. Or the opposite may be true where a soft sole shoe combined with softer surface running may lead to certain types of joint wear.

In some implementations, the user can indicate physical characteristics to provide better recommendations to the user. For example, the user can provide one or more of their weight, shoe size, pronation information (if known), biological gender, age, injury history, fitness level, or activity level (which may alternatively be supplied by a user profile automatically determined by the electronic device 130 and/or 132). These characteristics can be used to provide more accurate modelling. For example, one prediction model may be used for biological females versus biological males.

In some implementations, as discussed above, the signals can be provided to a companion device, such as a smart watch or phone. In some implementations, the signals can be filtered on the first and second device (e.g., the headphones) prior to sending to the companion device. The companion device may also be a tablet, wearable computerized eyeglasses, or a head mounted display.

In some implementations, at least a portion of the determining the one or more motion attributes of the user is performed on the companion device. For example, some of the data processing, such as noise reduction and signal filtering, may be performed on the first and second devices (e.g., the headphones), for example, by digital signal processing techniques, and the remainder of the processing may be performed on the companion device.

In some implementations, however, some of the processing on the first and second device may result in real-time feedback being supplied to the user. For example, a sound notification over the user's headphones can alert the user to check their phone or watch for details regarding the running dynamics estimation indicating a potential problem and suggestion for correction.

In some aspects, the process 600 can also include receiving information from the user related to an upcoming event, correlating the information to route information for the upcoming event, and suggesting a training route to the user based on the route information. For example, the user can supply information regarding an event the user is training for, such as a particular marathon. The electronic device 130 and/or 132 may obtain information for the upcoming event. For example, the electronic device 130 and/or 132 may retrieve route information for the event from the event organizer or from a server device that tracks and provides such information. The route information, route information can be analyzed by the electronic device 130 and/or 132 and a route in the immediate vicinity of the device can be suggested based on having similar characteristics. For example, if the route indicates uphill portions and downhill portions, hard surface and soft surfaces, the suggested route can attempt to match uphill and downhill portions, hard and soft surfaces as the route for the event. In some instances, the matching need not match the order of these features but may attempt to match the pitch and distance of a rise or fall, for example, and/or match a distance on grass or dirt versus on pavement. In some instances, the route information, for example, when not available for the upcoming event, may include historical route information for the event. For example, some events may have similar routes from year-to-year. In such instances, a historical route may be used to approximate the route of the upcoming event.

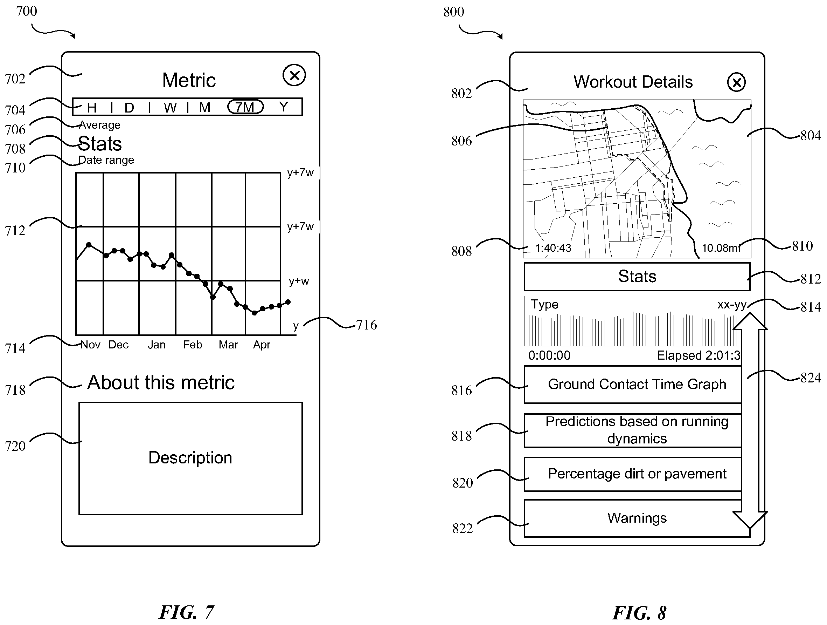

FIG. 7 illustrates a sample user interface 700 for displaying detailed information about a running dynamic estimate, for example determined by a running dynamic estimation using processes described herein, in accordance with some implementations. The interface is displayed as being an output to a smartphone, however, an output to any suitable electronic device, such as the electronic device 130 and/or 132 is contemplated.

Element 702 may correspond to a title for the particular running dynamic which estimate is shown. For example, the running dynamic may correspond to ground contact time. The name for the dynamic may be shown in the title section. Element 704 may correspond to a selection of a time period for which to show a graph of the data related to the running dynamic. For example, the time period can correspond to the past hour, day, week, month, 6-month period, or year, in this example. Other implementations can provide a control element to input a particular custom date range.

Element 706 indicates which kind of data is displayed for the element 708 for the selected time period. In the example, average data is displayed for the 6-month time period, however, any appropriate statistical data can be displayed, such as maximum, minimum, range, etc. Element 708 may correspond to a numeric value and unit for the statistical type specified in element 706. For example, in use, the word Stats may be replaced with a number such as 254 ms, indicating the average ground contact time over the entire six-month time period, for example. Element 710 can be used to show an actual data range for the six-month time period. For example, the graph indicated by element 712 may be horizontally scrollable and show a window having a length corresponding to the time period specified by element 704. Element 710 can provide the date range of the data displayed in the element 712.

Element 712 may correspond to a graph of the running dynamic displayed over the time period specified by element 704. The x-axis 714 may have labels based on both the date range and the time period and may adjust automatically according to the date range (element 710) and the time period (element 704). The y-axis 716 can show linear or logarithmic data. In the example illustrated in FIG. 7, the y-axis 716 is linear. The y-axis 716 may be graduated into values including y, y + w, y + 2w and y + 3w, or the like. The y-value may be selected based on the lowest value of the data in the graph and the w-value may be selected based on the maximum value of the data in the graph so as to display the extremes between the upper y-axis 716 value and the lower y-axis 716 value.

Element 718 may be a label for the box 720. For example, element 718 may display "about ground contact time" or another running dynamic. This about information may correspond to the title (element 702). Box 720 may contain a description of what the running dynamic means, including for example, units for the measurements of the particular running dynamic displayed.

It should be understood that the interface depicted in FIG. 7 is merely an example, which can be changed according to a designer's preferences. The graph (element 712) will change based on the underlying data and the selected values for the other elements.

FIG. 7 illustrates a sample user interface 800 for displaying workout details for a workout associated with a process of collecting sensor data and performing running dynamics estimation, in accordance with some implementations. The interface 800 is displayed as being an output to a smartphone, however, an output to any suitable electronic device, such as the electronic device 130 and/or 132 is contemplated.

Element 802 is a title for the page. In the example embodiment, the title is workout details, however, any suitable title may be used. Element 804 is a map of an area which is scaled appropriately to the route which was run. The map may include streets, names of places, satellite overlays, and so forth. Element 806 is an overlay on the map which shows the route which was run. Although demonstrated in dashed lines, the map may be displayed in color and the route overlaid in a particular color. The map (element 804) and route (element 806) may correspond to an event map for an upcoming event of the user. For example, the user may practice the actual route or may be provided a route that mimics at least some attributes of a route for an upcoming event, such as elevation changes, surface types, distances, and slopes. In some instances, the route may correspond to a historic route, for example, from a past event specified by the user. For example, suppose the user wants to run the 1995 Boston marathon, then the user can specify the event and the electronic device 130 and/or 132 can supply the route information, for example, by obtaining the route information from another source. A route for an upcoming event may similarly be obtained. Element 808 shows a time elapsed for the workout, and element 810 shows a distance elapsed for the workout.

Element 812 illustrates a menu item to select running dynamic estimates calculated for the workout. For example, element 812 may correspond to a stat for cadence. The graph 814 may illustrate an expanded view of a sub-menu item under the stats selection element 812. The graph 814, for example, may correspond to a visual representation of cadence estimates derived from the sensor data collected during the workout. The graph 814 may include an x-axis corresponding to the workout duration and a y-axis scaled to show data relevant to the metric being illustrated. The graph 814 may include a type value that says cadence in this example, and may further include an average or other statistic for the workout with a value and units for the value. For example, the label for the illustrated "Type" may be replaced with "Cadence average: 154 SPM," where SPM corresponds to steps per minute. The values xx-yy may be replaced with a range for the dynamic. In this example, they may be replaced with 122-175 SPM, for example.

Element 816 may correspond to a sub-menu item for the stats menu (element 814). If a user, for example, selects element 816, graph 814 may collapse, and element 816 may expand to show a graph similar to that displayed at graph 814. The collapsed graph 814 may then be replaced to display the label "Cadence," in this example.

Elements 818, 820, and 822 each may provide interface items, which when selected display corresponding data. For example, element 818, when selected may display predictions based on the running dynamics. These predictions may include predictions related to injury, for example, providing a prediction that an injury is developing or healing with respect to a user's foot or let. Such a prediction may be made, at least in part, by comparing the workout data to previous workout data and correlating that with running dynamics that indicate imbalance in right versus left foot and leg action. In some instances, the user may provide information regarding an injury, and that information can be used to help track healing of the injury. Predictions may also include those related to physical attributes of the user. For example, the predictions may include those related to whether the user has an over-pronated, under-pronated, or neutrally-pronated foot position when stepping. Also, the predictions may include those related to running technique, such as whether the user is a heel striker runner, a midfoot striker runner, or a front foot or ball striker runner. Such predictions may be made by supplying the sensor data to the models and classification systems trained to predict such data, such as described above.

Element 820 can provide information on what percentage the user's workout was on pavement versus dirt. It can also provide information on whether the whether the user's workout hit a target workout for percentage of pavement versus dirt and provide route suggestions to alter the workout to more closely achieve the user's desired distribution of surface type. Element 822 can provide warning information, such as information about where the running dynamics estimations show that the user needs improvement in their running style and or posture. Suggestions may also be provided to improve running dynamics, such as exercises or points of focus. Product suggestions such as footwear may also be suggested.

Element 824 indicates that the tiles corresponding to graph 814, element 816, element 818, element 820, and element 822 can be scrolled up and down, for example, to show additional running dynamics available to the user for display.

As described above, one aspect of the present technology is the gathering and use of data available from specific and legitimate sources for providing running dynamics estimations. The present disclosure contemplates that in some instances, this gathered data may include personal information data that uniquely identifies or can be used to identify a specific person. Such personal information data can include voice data, demographic data, location-based data, online identifiers, telephone numbers, email addresses, home addresses, data or records relating to a user's health or level of fitness (e.g., vital signs measurements, medication information, exercise information), date of birth, or any other personal information.

Some embodiments described herein can include use of learning and/or non-learning-based process(es). The use can include collecting, preprocessing, encoding, labeling, organizing, analyzing, recommending and/or generating data. Entities that collect, share, and/or otherwise utilize user data should provide transparency and/or obtain user consent when collecting such data. The present disclosure recognizes that the use of the data in the RDE processes can be used to benefit users. For example, the data can be used to train models that can be deployed to improve performance, accuracy, and/or functionality of applications and/or services. Accordingly, the use of the data enables the RDE processes to adapt and/or optimize operations to provide more personalized, efficient, and/or enhanced user experiences. Such adaptation and/or optimization can include tailoring content, recommendations, and/or interactions to individual users, as well as streamlining processes, and/or enabling more intuitive interfaces. Further beneficial uses of the data in the RDE processes are also contemplated by the present disclosure.

The present disclosure contemplates that, in some embodiments, data used by RDE processes includes publicly available data. To protect user privacy, data may be anonymized, aggregated, and/or otherwise processed to remove or to the degree possible limit any individual identification. As discussed herein, entities that collect, share, and/or otherwise utilize such data should obtain user consent prior to and/or provide transparency when collecting such data. Furthermore, the present disclosure contemplates that the entities responsible for the use of data, including, but not limited to data used in association with RDE processes, should attempt to comply with well-established privacy policies and/or privacy practices.

For example, such entities may implement and consistently follow policies and practices recognized as meeting or exceeding industry standards and regulatory requirements for developing and/or training RDE processes. In doing so, attempts should be made to ensure all intellectual property rights and privacy considerations are maintained. Training should include practices safeguarding training data, such as personal information, through sufficient protections against misuse or exploitation. Such policies and practices should cover all stages of the RDE processes development, training, and use, including data collection, data preparation, model training, model evaluation, model deployment, and ongoing monitoring and maintenance. Transparency and accountability should be maintained throughout. Such policies should be easily accessible by users and should be updated as the collection and/or use of data changes. User data should be collected for legitimate and reasonable uses of the entity and not shared or sold outside of those legitimate uses. Further, such collection and sharing should occur through transparency with users and/or after receiving the informed consent of the users. Additionally, such entities should consider taking any needed steps for safeguarding and securing access to such data and ensuring that others with access to the data adhere to their privacy policies and procedures. Further, such entities should subject themselves to evaluation by third parties to certify, as appropriate for transparency purposes, their adherence to widely accepted privacy policies and practices. In addition, policies and/or practices should be adapted to the particular type of data being collected and/or accessed and tailored to a specific use case and applicable laws and standards, including jurisdiction-specific considerations.

In some embodiments, RDE processes may utilize models that may be trained (e.g., supervised learning or unsupervised learning) using various training data, including data collected using a user device. Such use of user-collected data may be limited to operations on the user device. For example, the training of the model can be done locally on the user device so no part of the data is sent to another device. In other implementations, the training of the model can be performed using one or more other devices (e.g., server(s)) in addition to the user device but done in a privacy preserving manner, e.g., via multi-party computation as may be done cryptographically by secret sharing data or other means so that the user data is not leaked to the other devices.

In some embodiments, the trained model can be centrally stored on the user device (e.g., on a user's phone) or stored on multiple devices, e.g., as in federated learning, or in an ear bud. Such decentralized storage can similarly be done in a privacy preserving manner, e.g., via cryptographic operations where each piece of data is broken into shards such that no device alone (i.e., only collectively with another device(s)) or only the user device can reassemble or use the data. In this manner, a pattern of behavior of the user or the device may not be leaked, while taking advantage of increased computational resources of the other devices to train and execute the ML model. Accordingly, user-collected data can be protected. In some implementations, data from multiple devices can be combined in a privacy-preserving manner to train an ML model.

In some embodiments, the present disclosure contemplates that data used for RDE processes may be kept strictly separated from platforms where the RDE processes are deployed and/or used to interact with users and/or process data. In such embodiments, data used for offline training of the RDE processes may be maintained in secured datastores with restricted access and/or not be retained beyond the duration necessary for training purposes. In some embodiments, the RDE processes may utilize a local memory cache to store data temporarily during a user session. The local memory cache may be used to improve performance of the RDE processes. However, to protect user privacy, data stored in the local memory cache may be erased after the user session is completed. Any temporary caches of data used for online learning or inference may be promptly erased after processing. All data collection, transfer, and/or storage should use industry-standard encryption and/or secure communication.

In some embodiments, as noted above, techniques such as federated learning, differential privacy, secure hardware components, homomorphic encryption, and/or multi-party computation among other techniques may be utilized to further protect personal information data during training and/or use of the RDE processes. The RDE processes should be monitored for changes in underlying data distribution such as concept drift or data skew that can degrade performance of the RDE processes over time.

In some embodiments, the RDE processes are trained using a combination of offline and online training. Offline training can use curated datasets to establish baseline model performance, while online training can allow the RDE processes to continually adapt and/or improve. The present disclosure recognizes the importance of maintaining strict data governance practices throughout this process to ensure user privacy is protected.

In some embodiments, the RDE processes may be designed with safeguards to maintain adherence to originally intended purposes, even as the RDE processes adapt based on new data. Any significant changes in data collection and/or applications of RDE process use may (and in some cases should) be transparently communicated to affected stakeholders and/or include obtaining user consent with respect to changes in how user data is collected and/or utilized.

Despite the foregoing, the present disclosure also contemplates embodiments in which users selectively restrict and/or block the use of and/or access to data. That is, the present disclosure contemplates that hardware and/or software elements can be provided to prevent or block access to data. For example, in the case of some services, the present technology should be configured to allow users to select to "opt in" or "opt out" of participation in the collection of data during registration for services or anytime thereafter. In another example, the present technology should be configured to allow users to select not to provide certain data for training the RDE processes and/or for use as input during the inference stage of such systems. In yet another example, the present technology should be configured to allow users to be able to select to limit the length of time data is maintained or entirely prohibit the use of their data for use by the RDE processes. In addition to providing "opt in" and "opt out" options, the present disclosure contemplates providing notifications relating to the access or use of personal information. For instance, a user can be notified when their data is being input into the RDE processes for training or inference purposes, and/or reminded when the RDE processes generate outputs or make decisions based on their data.

The present disclosure recognizes RDE processes should incorporate explicit restrictions and/or oversight to mitigate against risks that may be present even when such systems having been designed, developed, and/or operated according to industry best practices and standards. For example, outputs may be produced that could be considered erroneous, harmful, offensive, and/or biased; such outputs may not necessarily reflect the opinions or positions of the entities developing or deploying these systems. Furthermore, in some cases, references to or failures to cite third-party products and/or services in the outputs should not be construed as endorsements or affiliations by the entities providing the RDE processes. Generated content can be filtered for potentially inappropriate or dangerous material prior to being presented to users, while human oversight and/or ability to override or correct erroneous or undesirable outputs can be maintained as a failsafe.

The present disclosure further contemplates that users of the RDE processes should refrain from using the services in any manner that infringes upon, misappropriates, or violates the rights of any party. Furthermore, the RDE processes should not be used for any unlawful or illegal activity, nor to develop any application or use case that would commit or facilitate the commission of a crime, or other tortious, unlawful, or illegal act including misinformation, disinformation, misrepresentations (e.g., deepfakes), deception, impersonation, and propaganda. The RDE processes should not violate, misappropriate, or infringe any copyrights, trademarks, rights of privacy and publicity, trade secrets, patents, or other proprietary or legal rights of any party, and appropriately attribute content as required. Further, the RDE processes should not interfere with any security, digital signing, digital rights management, content protection, verification, or authentication mechanisms. The RDE processes should not misrepresent machine-generated outputs as being human-generated.

Moreover, it is the intent of the present disclosure that personal information data should be managed and handled in a way to minimize risks of unintentional or unauthorized access or use. Risk can be minimized by limiting the collection of data and deleting data once it is no longer needed. In addition, and when applicable, including in certain health related applications, data de-identification can be used to protect a user's privacy. De-identification may be facilitated, when appropriate, by removing identifiers, controlling the amount or specificity of data stored (e.g., collecting location data at city level rather than at an address level), controlling how data is stored (e.g., aggregating data across users), and/or other methods such as differential privacy.

Therefore, although the present disclosure broadly covers use of personal information data to implement one or more various disclosed embodiments, the present disclosure also contemplates that the various embodiments can also be implemented without the need for accessing such personal information data. That is, the various embodiments of the present technology are not rendered inoperable due to the lack of all or a portion of such personal information data.

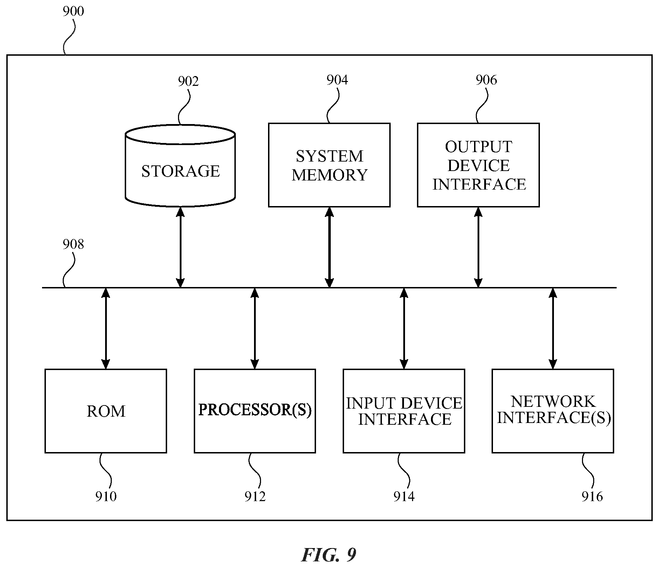

FIG. 9 illustrates an example computing device 900 with which aspects of the subject technology may be implemented in accordance with one or more implementations. The computing device 900 can be, and/or can be a part of, any computing device or server for generating the features and processes described above, including but not limited to a laptop computer, a smartphone, a tablet device, a wearable device such as a goggles or glasses, and the like. The computing device 900 may include various types of computer readable media and interfaces for various other types of computer readable media. The computing device 900 includes a permanent storage device 902, a system memory 904 (and/or buffer), an input device interface 906, an output device interface 908, a bus 910, a ROM 912, one or more processing unit(s) 914, one or more network interface(s) 916, and/or subsets and variations thereof.

The bus 910 collectively represents all system, peripheral, and chipset buses that communicatively connect the numerous internal devices of the computing device 900. In one or more implementations, the bus 910 communicatively connects the one or more processing unit(s) 914 with the ROM 912, the system memory 904, and the permanent storage device 902. From these various memory units, the one or more processing unit(s) 914 retrieves instructions to execute and data to process in order to execute the processes of the subject disclosure. The one or more processing unit(s) 914 can be a single processor or a multi-core processor in different implementations.

The ROM 912 stores static data and instructions that are needed by the one or more processing unit(s) 914 and other modules of the computing device 900. The permanent storage device 902, on the other hand, may be a read-and-write memory device. The permanent storage device 902 may be a non-volatile memory unit that stores instructions and data even when the computing device 900 is off. In one or more implementations, a mass-storage device (such as a magnetic or optical disk and its corresponding disk drive) may be used as the permanent storage device 902.

In one or more implementations, a removable storage device (such as a floppy disk, flash drive, and its corresponding disk drive) may be used as the permanent storage device 902. Like the permanent storage device 902, the system memory 904 may be a read-and-write memory device. However, unlike the permanent storage device 902, the system memory 904 may be a volatile read-and-write memory, such as random-access memory. The system memory 904 may store any of the instructions and data that one or more processing unit(s) 914 may need at runtime. In one or more implementations, the processes of the subject disclosure are stored in the system memory 904, the permanent storage device 902, and/or the ROM 912. From these various memory units, the one or more processing unit(s) 914 retrieves instructions to execute and data to process in order to execute the processes of one or more implementations.

The bus 910 also connects to the input and output device interfaces 906 and 908. The input device interface 906 enables a user to communicate information and select commands to the computing device 900. Input devices that may be used with the input device interface 906 may include, for example, alphanumeric keyboards and pointing devices (also called "cursor control devices"). The output device interface 908 may enable, for example, the display of images generated by computing device 900. Output devices that may be used with the output device interface 908 may include, for example, printers and display devices, such as a liquid crystal display (LCD), a light emitting diode (LED) display, an organic light emitting diode (OLED) display, a flexible display, a flat panel display, a solid state display, a projector, or any other device for outputting information.

One or more implementations may include devices that function as both input and output devices, such as a touchscreen. In these implementations, feedback provided to the user can be any form of sensory feedback, such as visual feedback, auditory feedback, or tactile feedback; and input from the user can be received in any form, including acoustic, speech, or tactile input.

Finally, as shown in FIG. 9, the bus 910 also couples the computing device 900 to one or more networks and/or to one or more network nodes through the one or more network interface(s) 916. In this manner, the computing device 900 can be a part of a network of computers (such as a LAN, a wide area network ("WAN"), or an Intranet, or a network of networks, such as the Internet. Any or all components of the computing device 900 can be used in conjunction with the subject disclosure.

Implementations within the scope of the present disclosure can be partially or entirely realized using a tangible computer-readable storage medium (or multiple tangible computer-readable storage media of one or more types) encoding one or more instructions. The tangible computer-readable storage medium also can be non-transitory in nature.

The computer-readable storage medium can be any storage medium that can be read, written, or otherwise accessed by a general purpose or special purpose computing device, including any processing electronics and/or processing circuitry capable of executing instructions. For example, without limitation, the computer-readable medium can include any volatile semiconductor memory, such as RAM, DRAM, SRAM, T-RAM, Z-RAM, and TTRAM. The computer-readable medium also can include any non-volatile semiconductor memory, such as ROM, PROM, EPROM, EEPROM, NVRAM, flash, nvSRAM, FeRAM, FeTRAM, MRAM, PRAM, CBRAM, SONOS, RRAM, NRAM, racetrack memory, FJG, and Millipede memory.

Further, the computer-readable storage medium can include any non-semiconductor memory, such as optical disk storage, magnetic disk storage, magnetic tape, other magnetic storage devices, or any other medium capable of storing one or more instructions. In one or more implementations, the tangible computer-readable storage medium can be directly coupled to a computing device, while in other implementations, the tangible computer-readable storage medium can be indirectly coupled to a computing device, e.g., via one or more wired connections, one or more wireless connections, or any combination thereof.

Instructions can be directly executable or can be used to develop executable instructions. For example, instructions can be realized as executable or non-executable machine code or as instructions in a high-level language that can be compiled to produce executable or non-executable machine code. Further, instructions also can be realized as or can include data. Computer-executable instructions also can be organized in any format, including routines, subroutines, programs, data structures, objects, modules, applications, applets, functions, etc. As recognized by those of skill in the art, details including, but not limited to, the number, structure, sequence, and organization of instructions can vary significantly without varying the underlying logic, function, processing, and output.

While the above discussion primarily refers to microprocessor or multi-core processors that execute software, one or more implementations are performed by one or more integrated circuits, such as ASICs or FPGAs. In one or more implementations, such integrated circuits execute instructions that are stored on the circuit itself.

Those of skill in the art would appreciate that the various illustrative blocks, modules, elements, components, methods, and algorithms described herein may be implemented as electronic hardware, computer software, or combinations of both. To illustrate this interchangeability of hardware and software, various illustrative blocks, modules, elements, components, methods, and algorithms have been described above generally in terms of their functionality. Whether such functionality is implemented as hardware or software depends upon the particular application and design constraints imposed on the overall system. Skilled artisans may implement the described functionality in varying ways for each particular application. Various components and blocks may be arranged differently (e.g., arranged in a different order, or partitioned in a different way) all without departing from the scope of the subject technology.

It is understood that any specific order or hierarchy of blocks in the processes disclosed is an illustration of example approaches. Based upon design preferences, it is understood that the specific order or hierarchy of blocks in the processes may be rearranged, or that all illustrated blocks be performed. Any of the blocks may be performed simultaneously. In one or more implementations, multitasking and parallel processing may be advantageous. Moreover, the separation of various system components in the implementations described above should not be understood as requiring such separation in all implementations, and it should be understood that the described program components and systems can generally be integrated together in a single software product or packaged into multiple software products.

As used in this specification and any claims of this application, the terms "base station", "receiver", "computer", "server", "processor", and "memory" all refer to electronic or other technological devices. These terms exclude people or groups of people. For the purposes of the specification, the terms "display" or "displaying" means displaying on an electronic device.

As used herein, the phrase "at least one of" preceding a series of items, with the term "and" or "or" to separate any of the items, modifies the list as a whole, rather than each member of the list (i.e., each item). The phrase "at least one of" does not require selection of at least one of each item listed; rather, the phrase allows a meaning that includes at least one of any one of the items, and/or at least one of any combination of the items, and/or at least one of each of the items. By way of example, the phrases "at least one of A, B, and C" or "at least one of A, B, or C" each refer to only A, only B, or only C; any combination of A, B, and C; and/or at least one of each of A, B, and C.

The predicate words "configured to", "operable to", and "programmed to" do not imply any particular tangible or intangible modification of a subject, but, rather, are intended to be used interchangeably. In one or more implementations, a processor configured to monitor and control an operation or a component may also mean the processor being programmed to monitor and control the operation or the processor being operable to monitor and control the operation. Likewise, a processor configured to execute code can be construed as a processor programmed to execute code or operable to execute code.

Phrases such as an aspect, the aspect, another aspect, some aspects, one or more aspects, an implementation, the implementation, another implementation, some implementations, one or more implementations, an embodiment, the embodiment, another embodiment, some implementations, one or more implementations, a configuration, the configuration, another configuration, some configurations, one or more configurations, the subject technology, the disclosure, the present disclosure, other variations thereof and alike are for convenience and do not imply that a disclosure relating to such phrase(s) is essential to the subject technology or that such disclosure applies to all configurations of the subject technology. A disclosure relating to such phrase(s) may apply to all configurations, or one or more configurations. A disclosure relating to such phrase(s) may provide one or more examples. A phrase such as an aspect or some aspects may refer to one or more aspects and vice versa, and this applies similarly to other foregoing phrases.

The word "exemplary" is used herein to mean "serving as an example, instance, or illustration". Any embodiment described herein as "exemplary" or as an "example" is not necessarily to be construed as preferred or advantageous over other implementations. Furthermore, to the extent that the term "include", "have", or the like is used in the description or the claims, such term is intended to be inclusive in a manner similar to the term "comprise" as "comprise" is interpreted when employed as a transitional word in a claim.

All structural and functional equivalents to the elements of the various aspects described throughout this disclosure that are known or later come to be known to those of ordinary skill in the art are expressly incorporated herein by reference and are intended to be encompassed by the claims. Moreover, nothing disclosed herein is intended to be dedicated to the public regardless of whether such disclosure is explicitly recited in the claims. No claim element is to be construed under the provisions of 35 U.S.C. § 112(f) unless the element is expressly recited using the phrase "means for" or, in the case of a method claim, the element is recited using the phrase "step for".

The previous description is provided to enable any person skilled in the art to practice the various aspects described herein. Various modifications to these aspects will be readily apparent to those skilled in the art, and the generic principles defined herein may be applied to other aspects. Thus, the claims are not intended to be limited to the aspects shown herein, but are to be accorded the full scope consistent with the language claims, wherein reference to an element in the singular is not intended to mean "one and only one" unless specifically so stated, but rather "one or more". Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the subject disclosure.

Exemplary embodiments of the present disclosure are set out in the following items:
1. A method, comprising:
   receiving a first signal from a first device within a first time period, wherein the first device is configured to be disposed on a first side of a user;
   receiving a second signal from a second device within the first time period, wherein the second device is configured to be disposed on a second side of the user, wherein the first device is configured to be in association with the second device;
   determining one or more motion attributes of the user based at least in part on the first signal in conjunction with the second signal, wherein the one or more motion attributes comprises a left-side or right-side specific motion attribute; and
   presenting feedback on a user device based at least in part on the one or more motion attributes.
2. The method of item 1, wherein the first signal comprises a signal from a first accelerometer or a first audio input, and wherein the second signal comprises a signal from a second accelerometer or a second audio input.
3. The method of item 2, further comprising:
   receiving a third signal from the first device within the first time period, the third signal comprising the other of the signal from the first accelerometer or the first audio input; and
   receiving a fourth signal from the second device within the first time period, the fourth signal comprising the other of the signal from the second accelerometer or the second audio input, wherein the determining the one or more motion attributes of the user is based further at least in part on the third signal in conjunction with the fourth signal.
4. The method of item 3, further comprising:
   weighting the first signal or third signal based on a sensed physical environment of the first device.
5. The method of any of items 1-4, further comprising:
   estimating one or more of cadence, ground contact time, ground contact time balance, stride length, or vertical oscillation based at least in part on the one or more motion attributes.
6. The method of item 5, further comprising:
   calculating embeddings based at least in part on the one or more motion attributes; and
   providing the embeddings to a classification model to determine the one or more estimations.
7. The method of any of items 5-6, wherein an estimation of ground contact time balance is used to determine heel strike information corresponding to a left foot of the user and heel strike information corresponding to a right foot of the user.
8. The method of any of items 1-7, wherein the determining comprises:
   generating an aggregate input from the first signal and the second signal during a first period of time, wherein the first signal and the second signal are filtered based on contextual information associated with pre-configured user settings; and
   determining, based on the aggregate input, one or more motion attributes during the first period of time.
9. A system, comprising:
   a processor; and
   a memory device, wherein the processor is configured to:
      receive a first signal from a first device within a first time period, wherein the first device is configured to be disposed on a first side of a user;
      receive a second signal from a second device within the first time period, wherein the second device is configured to be disposed on a second side of the user, wherein the first device is configured to be in association with the second device;
      determine one or more motion attributes of the user based at least in part on the first signal in conjunction with the second signal, wherein the one or more motion attributes comprises a left-side or right-side specific motion attribute; and
      provide feedback to a user device based at least in part on the one or more motion attributes.
10. The system of item 9, wherein the feedback includes an indication of detected right side or left side compensation.
11. The system of any of items 9-10, wherein the feedback includes an estimation of a percentage of time spent running on hard surfaces versus soft surfaces.
12. The system of any of items 9-11, wherein the processor is further configured to:
   receive user information for the user indicating physical characteristics of the user, wherein the physical characteristics include one or more of height, weight, injury history, fitness level, or activity level.
13. The system of any of items 9-12, wherein the processor is further configured to:
   receive information from the user related to an upcoming event;
   correlate the information to route information for the upcoming event; and
   suggest a training route to the user based on the route information.
14. The system of item 13, wherein the route information includes historical route information for a previous version of the upcoming event.
15. The system of any of items 9-14, wherein the first signal comprises a first accelerometer signal, and wherein the second signal comprises a second accelerometer signal, wherein the processor is further configured to:
   receive a third signal comprising a first audio signal within the first time period;
   receive a fourth signal comprising a second audio input within the first time period; and
   weighting the first signal or third signal based on a sensed physical environment of the first device, wherein the determining the one or more motion attributes of the user is based further at least in part on the third signal in conjunction with the fourth signal.
16. A non-transitory computer-readable medium storing instructions thereon, which when executed by one or more processors, cause the one or more processors to:
   receive a first signal from a first device within a first time period, wherein the first device is configured to be disposed on a first side of a user;
   receive a second signal from a second device within the first time period, wherein the second device is configured to be disposed on a second side of the user, wherein the first device is configured to be in association with the second device;
   determine one or more motion attributes of the user based at least in part on the first signal in conjunction with the second signal, wherein the one or more motion attributes comprises a left-side or right-side specific motion attribute; and
   provide feedback to a user device based at least in part on the one or more motion attributes.
17. The non-transitory computer-readable medium of item 16, wherein the instructions further cause the one or more processors to:
   filtering, on the first device, the first signal;
   filtering, on the second device, the second signal; and
   providing a sample of each signal to a companion device associated with the first device and the second device.
18. The non-transitory computer-readable medium of item 17, wherein at least a portion of the determining the one or more motion attributes of the user is performed on the companion device.
19. The non-transitory computer-readable medium of any of items 17-18, wherein the instructions further cause the one or more processors to:
   provide at least some of the determining the one or more motion attributes of the user on the first device or on the second device; and
   provide real-time feedback to the user.
20. The non-transitory computer-readable medium of any of items 16-19, wherein the first signal is from a first in-ear microphone and wherein the second signal is from a second in-ear microphone.

## Claims

1. A method, comprising:
receiving a first signal from a first device within a first time period, wherein the first device is configured to be disposed on a first side of a user;
receiving a second signal from a second device within the first time period, wherein the second device is configured to be disposed on a second side of the user, wherein the first device is configured to be in association with the second device;
determining one or more motion attributes of the user based at least in part on the first signal in conjunction with the second signal, wherein the one or more motion attributes comprises a left-side or right-side specific motion attribute; and
presenting feedback on a user device based at least in part on the one or more motion attributes.

2. The method of claim 1, wherein the first signal comprises a signal from a first accelerometer or a first audio input, and wherein the second signal comprises a signal from a second accelerometer or a second audio input, wherein the method further comprises:
receiving a third signal from the first device within the first time period, the third signal comprising the other of the signal from the first accelerometer or the first audio input; and
receiving a fourth signal from the second device within the first time period, the fourth signal comprising the other of the signal from the second accelerometer or the second audio input, wherein the determining the one or more motion attributes of the user is based further at least in part on the third signal in conjunction with the fourth signal.

3. The method of claim 2, further comprising:
weighting the first signal or third signal based on a sensed physical environment of the first device.

4. The method of any of claims 1-3, further comprising:
estimating one or more of cadence, ground contact time, ground contact time balance, stride length, or vertical oscillation based at least in part on the one or more motion attributes.

5. The method of claim 4, further comprising:
calculating embeddings based at least in part on the one or more motion attributes; and
providing the embeddings to a classification model to determine the one or more estimations.

6. The method of any of claims 4-5, wherein an estimation of ground contact time balance is used to determine heel strike information corresponding to a left foot of the user and heel strike information corresponding to a right foot of the user.

7. The method of any of claims 1-6, wherein the determining comprises:
generating an aggregate input from the first signal and the second signal during a first period of time, wherein the first signal and the second signal are filtered based on contextual information associated with pre-configured user settings; and
determining, based on the aggregate input, one or more motion attributes during the first period of time.

8. The method of any of claims 1-7, wherein the feedback includes an indication of detected right side or left side compensation.

9. The method of any of claims 1-8, wherein the feedback includes an estimation of a percentage of time spent running on hard surfaces versus soft surfaces.

10. The method of any of claims 1-9, further comprising:
receiving information from the user related to an upcoming event;
correlating the information to route information for the upcoming event; and
suggesting a training route to the user based on the route information.

11. The method of any of claims 1-10, further comprising:
filtering, on the first device, the first signal;
filtering, on the second device, the second signal; and
providing a sample of each signal to a companion device associated with the first device and the second device.

12. The method of claim 11, wherein at least a portion of the determining the one or more motion attributes of the user is performed on the companion device.

13. The method of any of claims 1112, further comprising,
providing at least some of the determining the one or more motion attributes of the user on the first device or on the second device; and
providing real-time feedback to the user.

14. A system, comprising:
a processor; and
a memory device, wherein the processor is configured to perform operations comprising any of claims 1-13.

15. A non-transitory computer-readable medium storing instructions thereon, which when executed by one or more processors, cause the one or more processors to perform operations comprising any of claims 1-13.
